Europäisches Patentamt

(19) European Patent Office (11) Publication number: **0 108 173**
Office européen des brevets **B1**

(12) **EUROPEAN PATENT SPECIFICATION**

(45) Date of publication of patent specification: **14.05.86** (51) Int. Cl.⁴: **A 41 B 13/02**

(21) Application number: **82305800.3**

(22) Date of filing: **01.11.82**

(54) Method and apparatus for applying elastic bands to webs.

<table>
<tr><td>(43) Date of publication of application:<br>**16.05.84 Bulletin 84/20**</td><td>(73) Proprietor: **Sabee, Reinhardt Nils**<br>**728 South Summit Street**<br>**Appleton Wisconsin 54911 (US)**</td></tr>
<tr><td>(45) Publication of the grant of the patent:<br>**14.05.86 Bulletin 86/20**</td><td>(72) Inventor: **Sabee, Reinhardt Nils**<br>**728 South Summit Street**<br>**Appleton Wisconsin 54911 (US)**</td></tr>
<tr><td>(84) Designated Contracting States:<br>**DE FR GB SE**</td><td rowspan="2">(74) Representative: **Fisher, Bernard et al**<br>**Raworth, Moss & Cook 36 Sydenham Road**<br>**Croydon Surrey CR0 2EF (GB)**</td></tr>
<tr><td>(56) References cited:<br>**EP-A-0 047 106**<br>**EP-A-0 048 011**<br>**US-A-4 081 301**<br>**US-A-4 171 239**<br>**US-A-4 227 952**</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

This invention relates to a method and apparatus for applying elastic bands to webs.

Various techniques have been developed for applying elastic ribbons to webs to provide an elasticized leg portion in a disposable diaper. U.S.—A—4,081,301; U.S.—A—4,239,578 and U.S.—A—4,227,952 and the patents cited therein are illustrative of the known methods.

The invention is of application to the manufacture of disposable diapers with elasticized legs contacting portions in the diaper and more specifically to method and apparatus for applying the elastic bands to the backing or facing sheet. The difficulties in prior art methods, including handling discrete lengths of elastic ribbon with adhesive applied are, at least to a large extent, overcome by the method and apparatus of the present invention.

According to the present invention there is provided a method of intermittently attaching lengths of stretched elastic ribbon to spaced portions of a substantially non-elsatic moving web in which adhesive is applied to discrete portions of the stretched elastic ribbon, the stretched ribbon and moving web are fed to a station for applying the ribbon to the web and at least a portion of the ribbon is maintained in the stretched condition until the adhesive sets to adhere isolated portions of stretched ribbon to the web, the ribbon and the web being supported on a base surface at the elastic ribbon applying station characterised in that a non-adhered portion of the ribbon is severed by pressing cutting means simultaneously against the ribbon and a portion of the width of the web without severing the web.

The invention also provides an apparatus for intermittently attaching lengths of stretched elastic ribbon to spaced portions of a substantially non-elastic moving web comprising means operable to apply adhesive to discrete portions of the stretched elastic ribbon, means operable to feed the stretched ribbon and moving web to a station for applying the ribbon to the web and means for maintaining at least a portion of the ribbon in the stretched condition until the adhesive sets to adhere isolated portions of stretched ribbon to the web, a base surface being provided at the elastic ribbon applying station characterised by cutting means operable to press simultaneously against the ribbon and a portion of the width of the web without severing the web.

The elastic ribbons are held on the base surface, which in the preferred embodiment is the periphery of a rotating drum by various techniques including vacuum and mechanical gripping devices or its own tension or the tension of facing and backing webs. The elastic bands are cut into the desired length either before or after the backing or facing web to which the elastic bands are adhered is fed to the drum supporting surface. Alternately, the supporting surface can be a series of interconnected plates.

The cut off of the elastic bands to the desired length is accomplished by knives and cooperating anvil rolls or anvil bars, with either the anvil bar or knife located on the drum and the other cooperating part located on a rotating wheel located close to the drum supporting surface.

After the web with adhered elastic ribbons leaves the drum, absorbent pads and a second web, which can be the other of a facing or backing sheet, can be applied and the composite assembly cut off to form individual diapers.

In some embodiments the belt tension of an auxiliary belt having a run wrapped around the periphery of the drum may be employed to maintain tension or pressure on the elastic bands and the web backing or facing until the adhesive sets.

Embodiments of the invention will now be described by way of example, reference being made to the accompanying drawings in which:—

Fig. 1 is a diagrammatic sectional view of an embodiment of the invention;

Fig. 1A is an edge view of a web with applied band;

Fig. 2 is a diagrammatic perspective view of an alternate embodiment of the invention;

Fig. 3 is an edge view of the plastic band and backing or facing sheet laminate with the ends relaxed;

Fig. 4 is a diagrammatic sectional view of a further embodiment of the invention;

Fig. 5 is a diagrammatic view of a further embodiment of the invention;

Fig. 6 is a fragmentary view of an apparatus similar to Fig. 5 in which the web overlies the ribbon;

Fig. 7 is a diagrammatic edge view of elastic band applied to the web by apparatus shown in Fig. 4;

Fig. 8 is a view similar to Fig. 7 with the glue applied intermittently in a pattern;

Fig. 9 is a diagrammatic view similar to Fig. 1A with the glue applied intermittently in a pattern;

Fig. 10 is a view similar to Fig. 7 with the glue applied differently; and

Fig. 11 is a view similar to Fig. 7 with the glue applied in a further pattern.

Although the disclosure hereof is detailed and exact to enable those skilled in the art to practice the invention, the physical embodiments herein disclosed merely exemplify the invention which may be embodied in other specific structure. The scope of the invention is defined in the claims appended hereto.

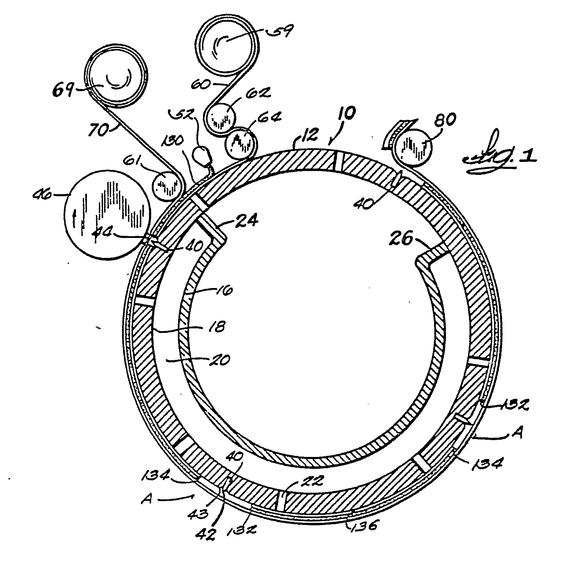

Fig. 1 shows apparatus used for practicing the invention which includes a rotatable drum 10 having a peripheral surface 12 which serves as the base surface support or working surface for accomplishing the application of adhesive to an elastic band, cutting the elastic band to length and assembling the elastic bands, the backing or facing sheet. The drum includes wall portions 16 and 18 which define a toroidal vacuum chamber 20 which communicates with the periphery 12 through vacuum passages 22. The vacuum passages 22 communicate with the portion of arc of travel of the drum between end walls 24 and 26.

The drum structure illustrated in Fig. 1 also includes a plurality of fixed knives 40 which are circumferentially arranged about the drum and project in a radial direction outwardly with respect to the axis of the drum. The knives have a cutting edge 42 which projects beyond the periphery of the drum and extends into a cavity or recess 43 in the periphery 12 of the drum. The knives 40 cooperate with anvil bars 44 carried by rotating anvil bar holder 46 which rotates in timed sequence to position an anvil bar 44 to coact with the knives 40 as the recesses move past the anvil holder 46. Inasmuch as two elastic bands are employed, the assembly shown in Fig. 1 only represents the equipment used to handle one elastic band. Duplicate equipment would be at a point spaced axially of the drum from that shown in Fig. 1.

An adhesive applicator 52 applies beads of adhesive to the elastic ribbons.

In use, pairs of elastic bands or ribbons 60 are drawn from parent rollers 59 and applied in an axially spaced arrangement on the drum by guide rollers 62 and 64. Rollers 64 apply the elastic ribbon 60 under tension.

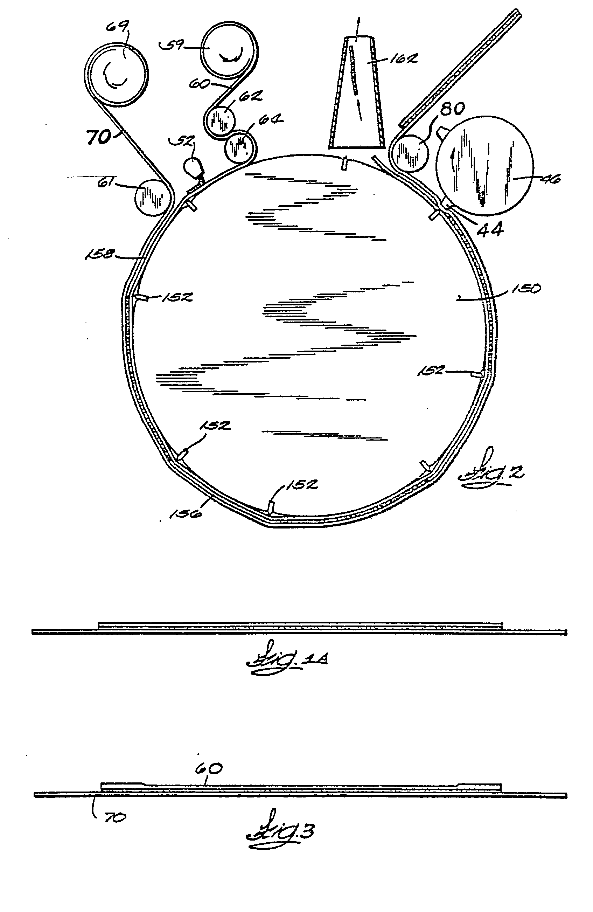

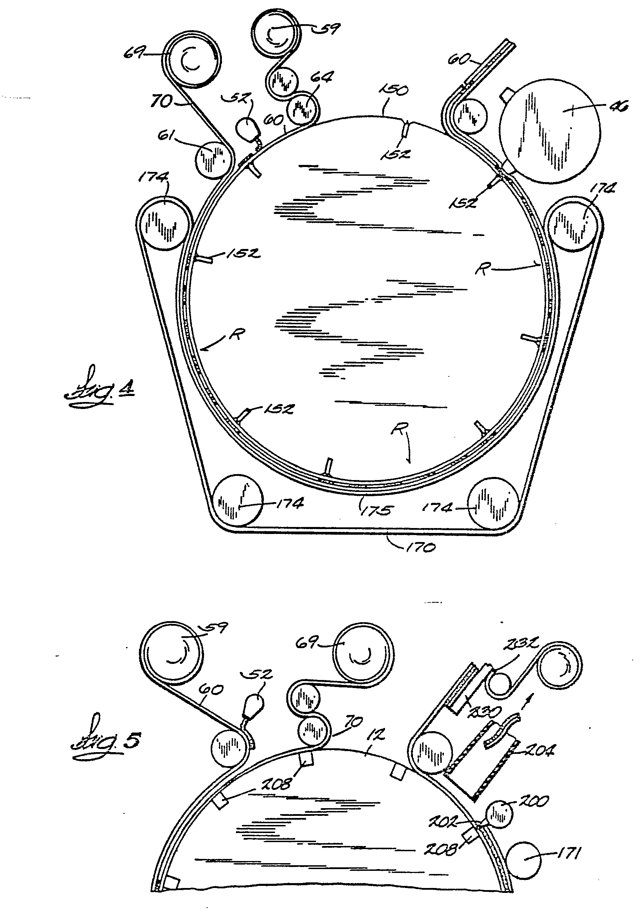

Vacuum gripping combined with tension of backing or facing webs is employed to maintain the ribbon or elastic band on the periphery of the drum. In the structure disclosed, the knives 40 cooperate with anvil bars 44 to sever the elastic ribbon while under tension. The anvil roll and knives perform the cut on the spaced elastic ribbons after the web has been applied. The plastic backing or facing sheet or web 70 is drawn from a parent roll 69 and a feed roll 61 pinches the applied backing or facing web 70 against the elsatic band segment 130 to maintain tension between the web feed roll 61 and the ribbon feed roll 64. When the cut has been made after the web is applied, the ends 132 and 134 of the elastic bands relax and become thicker, as shown in Fig. 3, because the adhesive is not yet set and, as shown at point A on the drum periphery, the relaxed ends 132 and 134 have separated and, although they are adhesively adhered, there is no tension at these ends. The vacuum ducts or suction passages 22, however, secure the tensioned elastic ribbon to the drum periphery to maintain tension on the ribbon segment 136 in between the relaxed ends. The gap between the ends 132 and 134 becomes the waistband portion of the diaper as can be seen in Fig. 1A and Fig. 3. The web with the adhered elastic band leaves the periphery of the drum over roller 80.

Fig. 2 shows a drum 150 with a plurality of spaced knives 152 and a web feed roll 69 and elastic band feed roll 59. The anvil roller 46 in this case causes cut off of the elastic ribbon after the adhesive has set and just prior to removal of the web and applied adhesive band assembly from the drum over roller 80. The adhesive is applied to discrete portions of the stretched elastic ribbon 60 by applicators 52 so that there are areas 156 and 158 of the ribbon 60 between those portions to which adhesive has been applied which areas 156 and 158 do not adhere to the web and provide waistband portions which do not have elastic bands as shown in Fig. 1A. Tension of the elastic ribbon on the drum holds the elastic bands in place until the glue has set and the cut off is made. The non-adhered portions 156, 158 of the elastic band can be removed by a suction removal device such as that shown at 162. The adhesive can be applied intermittently to those portions of the stretched ribbon to be adhered to the web, e.g., in the form of a series of dots.

Fig. 3 shows a section of the web and ribbon assembly produced by the method of an apparatus shown in Fig. 1.

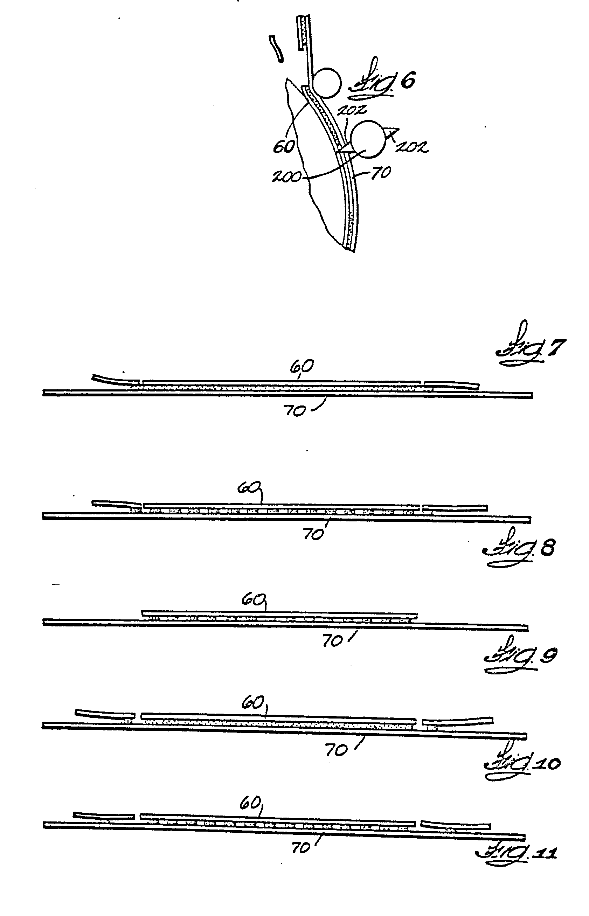

The embodiment illustrated in Fig. 4 is similar to that illustrated in Fig. 2. In this case, the drum 150 employs an endless tension belt 170 trained around rollers 174, which has a working run 175 pressed against the drum 150, to provide tension to maintain the stretched condition of the elastic ribbon 60 until the adhesive supplied by applicator 52 sets. The adhesive can be applied continuously at spaced locations to those lengths of the elastic ribbon to be adhered as shown at zone R. The absence of adhesive between such lengths will result in separated independent relaxed segments at each end of the adhered portions when the plastic ribbon is cut by cooperation of the anvil roller 46 and cutting knives 152.

Fig. 5 illustrates a further embodiment of the invention. In this embodiment the film or web 70, either backing or facing material, lies between the elastic ribbon 60 and the drum surface 12. Adhesive is provided by applicator 52 to the elastic ribbon prior to deposit of the ribbon on the film. A squeeze roll 171 is employed to maintain tension and hold the elastic band against the film until the adhesive sets. The adhesive is provided intermittently to provide lengths of elastic ribbon having areas where there is no glue and such areas of the elastic band are severed from the adhered portions by a rotary cut-off knife 200 which has one or more cutting blades 202 which pinch against the elastic ribbon which is under tension and which may or may not provide a small nick or slit in the under-lying plastic film. The plastic backing does not provide any waterproofing function at this location in a diaper and a mark or even a small slit is immaterial with respect to the functioning of the finished product. Cutting the ribbon against the plastic film simplifies the apparatus considerably and allows much higher speeds than could be obtained otherwise. The severed elastic ribbon sections can be taken away from the working area by a vacuum assist 204. The drum periphery 12 can be provided with spaced anvil bars 208, as illustrated. Alternatively, the knife can pinch against the periphery 12 of the drum, the latter providing the anvil surface.

Fig. 6 shows a modification of Fig. 5. In this arrangement the film 70 overlies the elastic ribbon 60 and the knife 202 pierces the backing or facing material 70 with a small slit prior to cutting the non-adhered portion of the elastic ribbon 60 from the ribbon/web assembly. Again, the slit in the backing or facing sheet 70 would not be in a zone which is subject to leakage. Of the various embodiments illustrated herein, it is believed at this time that the apparatus illustrated in Figs. 2, 5 and 6 are the preferred embodiments for high speed operation.

Figs. 7 through 11 illustrate various combinations of dots, dashes and spaces which may be obtained in an adhesive pattern by adjusting the timing of the adhesive applicator. As is apparent, products may be produced with no unadhered ends, with unadhered ends of equal or unequal lengths or relaxed adhered ends of the elastic ribbon to the underlying web.

As illustrated in Fig. 5, after the elastic ribbon and web is withdrawn from the drum, absorbent pads 230 can be applied and the other of the facing and backing sheets 232 can be superimposed.

It will be appreciated from the described embodiments that elastic ribbon is severed after application to the web and whilst the web and the rib are supported on the support or base surface provided by the drum. This simplifies the apparatus considerably and allows much higher speeds of manufacture to be obtained than would otherwise be achieved. The elastic ribbon is severed by cutting means which press simultaneously on the ribbon and the web. In the embodiments of Figs. 1, 2, 4 and 5, the cutting knife is applied to the ribbon as the web is supported on an anvil surface. The web may be marked or penetrated but this only occurs at a location which is not required to provide any waterproofing function in the eventual diaper and is therefore acceptable. Similar comments apply in the case of the Fig. 6 embodiment although in this case, the cutting knife is applied to the web and penetrates the web to cut and sever the ribbon.

It is to be understood that the cutting edge of the knives or blades described is relatively narrow and only slightly wider than the elastic ribbon to ensure that the ribbon is severed and any consequent penetration or cut in the web is of a limited extent which, as explained above, is acceptable and the web is not severed.

In the above described embodiments various means are described for holding the stretched elastic band or ribbon on the base surface support provided by the drum. The described means include vacuum means, a separate tension band and also utilising the web applied over the stretched elastic ribbon. Other means can be employed. For example, anchoring pins mounted on the drum and associated with the cutting means may be employed to engage the stretched elastic ribbon as it is fed to and applied on the drum.

## Claims

1. A method of intermittently attaching lengths of stretched elastic ribbon to spaced portions of a substantially non-elastic moving web in which adhesive is applied to discrete portions of the stretched elastic ribbon, the stretched ribbon and moving web are fed to a station for applying the ribbon to the web and at least a portion of the ribbon is maintained in the stretched condition until the adhesive sets to adhere isolated portions of stretched ribbon to the web, the ribbon and the web being supported on a base surface at the elastic ribbon applying station characterised in that a non-adhered portion of the ribbon is severed by pressing cutting means simultaneously against the ribbon and a portion of the width of the web without severing the web.

2. A method according to claim 1 characterised in that the elastic ribbon is held on the base surface under tension by a vacuum.

3. A method according to claim 1 or 2 characterised in that the web overlies the ribbon on the base surface and at least in part assists in retaining the ribbon under tension.

4. A method according to claim 1, 2 or 3 characterised in that the ribbon and the web are held against the base surface by a tension belt.

5. A method according to any one of the preceding claims characterised in that the ribbon and the web are fed to a rotatable drum so that one overlies the other and are passed between cutting means comprising a cutting edge coacting with an anvil surface one of which is associated with the base surface which is associated with the drum periphery.

6. A method according to claim 5 characterised in that the web overlies the ribbon and the cutting edge is associated with the base surface to engage the ribbon and coact with the anvil surface engaged with the web.

7. A method according to claim 5 characterised in that the web overlies the ribbon, the anvil surface is associated with the base surface to engage the ribbon and the cutting edge is operable to penetrate the web and coact with the anvil surface to sever the ribbon.

8. A method according to claim 5 characterised in that the ribbon overlies the web, the anvil surface is associated with the base surface to engage the web and the cutting edge is operable to engage and sever the ribbon.

9. A method according to any one of the preceding claims characterised in that the ribbon severing step forms one of a slit and a mark on the web.

10. A method according to any one of the preceding claims characterised by the steps of applying an absorbent pad to the web with the ribbon adhered thereto, applying a backing or facing sheet over the pad and cutting individual diapers from the assembled web.

11. An apparatus for intermittently attaching lengths of stretched elastic ribbon to spaced portions of a substantially non-elastic moving web comprising means operable to apply adhesive to discrete portions of the stretched elastic ribbon, means operable to feed the stretched ribbon and moving web to a station for applying the ribbon to the web and means for maintaining at least a portion of the ribbon in the stretched condition until the adhesive sets to adhere isolated portions of stretched ribbon to the web, a base surface being provided at the elastic ribbon applying station characterised by cutting means operable to press simultaneously against the ribbon and a portion of the width of the web without severing the web.

12. Apparatus according to claim 11 characterised in that the base surface is associated with the periphery of a rotatable drum to receive the ribbon and the web in superposed relationship and the cutting means comprises a cutting edge and an anvil surface arranged in coacting relationship, one of the cutting edge and the anvil surface being associated with the base surface.

13. Apparatus according to claim 12 characterised in that the cutting edge is associated with the base surface for rotation with the drum and the anvil surface is arranged to coact with the cutting edge as the superposed ribbon and web pass therebetween.

14. Apparatus according to claim 12 characterised in that the anvil surface is associated with the base surface for rotation with the drum and the cutting edge is arranged to coact with the anvil surface as the superposed ribbon and web pass therebetween.

**Patentansprüche**

1. Verfahren zum intermittierenden Anbringen von Längen von gedehntem elastischem Band an beabstandete Abschnitte einer im wesentlichen nicht elastischen, sich bewegenden Bahn, bei dem Kleber auf diskrete Abschnitte des gedehnten elastischen Bandes aufgebracht wird, das gedehnte Band und die sich bewegende Bahn einer Station zur Anbringung des Bandes an die Bahn zugeführt werden und mindestens ein Abschnitt des Bandes in dem gedehnten Zustand gehalten wird, bis der Kleber abbindet, um isolierte Abschnitte des gedehnten Bandes an der Bahn zu befestigen, wobei das Band und die Bahn auf einer Basisoberfläche bei der Anbringstation für das elastische Band gehaltert werden, dadurch gekennzeichnet, daß ein nicht befestigter Abschnitt des Bandes dadurch abgetrennt wird, daß Schneidmittel gleichzeitig gegen das Band und einen Abschnitt der Breite der Bahn gepreßt werden, ohne die Bahn zu zertrennen.

2. Verfahren nach Anspruch 1, dadurch gekennzeichnet, daß das elastische Band auf der Basisoberfläche durch ein Vakuum unter Spannung gehalten wird.

3. Verfahren nach Anspruch 1 oder 2, dadurch gekennzeichnet, daß die Bahn über dem Band auf der Basisoberfläche liegt und mindestens teilweise mithilft, das Band unter Spannung zu halten.

4. Verfahren nach Anspruch 1, 2 oder 3, dadurch gekennzeichnet, daß das Band und die Bahn durch einen Spannungsriemen gegen die Basisoberfläche gehalten werden.

5. Verfahren nach einem der vorhergehenden Ansprüche, dadurch gekennzeichnet, daß das Band und die Bahn einer drehbaren Trommel derart zugeführt werden, daß eines über dem anderen liegt, und zwischen Schneidmitteln hindurchgeführt werden, die eine mit einer Amboßoberfläche zusammenwirkende Schneidkante enthalten, von denen ein Element der Basisoberfläche zugeordnet ist, die dem Trommelumfang zugeordnet ist.

6. Verfahren nach Anspruch 5, dadurch gekennzeichnet, daß die Bahn über dem Band liegt und die Schneidkante der Basisoberfläche zum Angriff an dem Band und zur Zusammenwirkung mit der Amboßoberfläche zugeordnet ist, die an der Bahn angreift.

7. Verfahren nach Anspruch 5, dadurch gekennzeichnet, daß die Bahn über dem Band liegt, die Amboßoberfläche der Basisoberfläche zum Angriff an dem Band zugeordnet ist und die Schneidkante derart betätigbar ist, daß sie die Bahn durchdringt und mit der Amboßoberfläche zum Abtrennen des Bandes zusammenwirkt.

8. Verfahren nach Anspruch 5, dadurch gekennzeichnet, daß das Band über der Bahn liegt, die Amboßoberfläche der Basisoberfläche zum Angriff an der Bahn zugeordnet ist und die Schneidkante zum Angriff und zum Durchtrennen des Bandes betätigbar ist.

9. Verfahren nach einem der vorhergehenden Ansprüche, dadurch gekennzeichnet, daß der Verfahrensschritt des Durchtrennens des Bandes einen Schlitz oder eine Kerbe an der Bahn bildet.

10. Verfahren nach einem der vorhergehenden Ansprüche, dadurch gekennzeichnet, daß ein absorbierendes Kissen an der Bahn mit dem daran anhaftenden Band angebracht wird, ein hinterer oder vorderer Streifen über dem Kissen angebracht und einzelne Windeln von der zusammengesetzten Bahn geschnitten werden.

11. Vorrichtung zum intermittierenden Anbringen von Längen von gedehntem elastisches Band an beabstandeten Abschnitten einer im wesentlichen nicht elastischen, sich bewegenden Bahn, mit Mitteln zum Aufbringen von Kleber auf diskrete Abschnitte des gedehnten elastischen Bandes, Mitteln zum Zuführen des gedehnten Bandes und der sich bewegenden Bahn zu einer Station zur Anbringung des Bandes an der Bahn sowie Mitteln, um mindestens einen Abschnitt des Bandes in der gedehnten Stellung zu halten, bis der Kleber abbindet, um isolierte Abschnitte gedehnten Bandes an der Bahn zu befestigen, wobei an der Anbringstation des elastischen Bandes eine Basisoberfläche vorgesehen ist, gekennzeichnet durch Schneidmittel, die derart betätigbar sind, daß sie gleichzeitig gegen das Band und einen Abschnitt der Breite der Bahn drücken, ohne die Bahn zu zertrennen.

12. Vorrichtung nach Anspruch 11, dadurch gekennzeichnet, daß die Basisoberfläche dem Umfang einer drehbaren Trommel zur Aufnahme des Bandes und der Bahn in übereinanderliegender Beziehung zugeordnet ist und die Schneidmittel eine in zusammenwirkender Beziehung angeordnete Schneidkante und Amboßoberfläche aufweisen, wobei die Schneidkante oder die Amboßoberfläche der Basisoberfläche zugeordnet ist.

13. Vorrichtung nach Anspruch 12, dadurch gekennzeichnet, daß die Schneidkante der Basisoberfläche zur Drehung mit der Trommel zugeordnet und die Amboßoberfläche zum Zusammenwirken mit der Schneidkante angeordnet ist, wenn Band und Bahn übereinanderliegend zwischen ihnen hindurchgehen.

14. Vorrichtung nach Anspruch 12, dadurch gekennzeichnet, daß die Amboßoberfläche der Basisoberfläche zur Drehung mit der Trommel zugeordnet und die Schneidkante zur Zusammenwirkung mit der Amboßoberfläche angeordnet ist, wenn Band und Bahn übereinanderliegend zwischen ihnen hindurchgehen.

**Revendications**

1. Procédé pour appliquer, de façon intermittente, des longueurs de rubans élastiques étirés sur des portions espacées d'une bande sensiblement non élastique en déplacement, selon lequel de l'adhésif est appliqué à des portions discrètes du ruban élastiques étiré, le ruban étiré et la bande en déplacement étant délivrés à un poste d'application du ruban sur la bande, et une portion au moins du ruban étant maintenue à l'état étiré jusqu'à ce que l'adhésif durcisse, afin de faire adhérer des portions isolées du ruban étiré sur la bande, le ruban et la bande étant supportés par une surface de base au poste d'application du ruban élastique, caractérisé en ce qu'on découpe une portion du ruban n'ayant pas adhéré, en appliquant simultanément des moyens de coupe contre le ruban et une partie de la largeur de la bande, sans couper la bande.

2. Procédé selon la revendication 1, caractérisé en ce que le ruban élastique est maintenu sur la surface de base sous tension par l'action d'un vide.

3. Procédé selon la revendication 1 ou 2, caractérisé en ce que la bande recouvre le ruban sur la surface de base et aide, au moins en partie, à maintenir le ruban sous tension.

4. Procédé selon la revendication 1, 2 ou 3, caractérisé en ce que le ruban et la bande sont maintenus contre la surface de base à l'aide d'une courroie sous tension.

5. Procédé selon l'une quelconque des revendications précédentes, caractérisé en ce que le ruban et la bande sont délivrés à un tambour rotatif de manière que l'un recouvre l'autre, et ils passent entre des moyens de coupe qui comprennent une arête tranchante coopérant avec une surface de billot, l'un de ces éléments étant associé à la surface de base associée à la périphérie du tambour.

6. Procédé selon la revendication 5, caractérisé en ce que la bande recouvre le ruban, et l'arête tranchante est associée à la surface de base de manière à venir en prise avec le ruban et à coopérer avec la surface du billot en prise avec la bande.

7. Procédé selon la revendication 5, caractérisé en ce que la bande recouvre le ruban, la surface du billot est associée à la surface de base afin de venir en prise avec le ruban, et l'arête tranchante fonctionne de façon à pénétrer la bande et à coopérer avec la surface du billot pour découper le ruban.

8. Procédé selon la revendication 5, caractérisé en ce que le ruban recouvre la bande, la surface du billot est associée à la surface de base afin de venir en prise avec la bande, et l'arête tranchante fonctionne de manière à venir en prise avec le ruban et à découper ce dernier.

9. Procédé selon l'une quelconque des revendications précédentes, caractérisé en ce que l'étape de coupe du ruban forme une fente ou une marque sur la bande.

10. Procédé selon l'une quelconque des revendications précédentes, caractérisé par les étapes qui consistent à appliquer un tampon absorbant sur la bande, le ruban adhérant à cette dernière, à appliquer une feuille dorsale ou frontale sur le tampon, et à découper des motifs individuels à partir de la bande assemblée.

11. Dispositif pour appliquer, de façon intermittente, des longueurs de rubans élastiques étirés sur des portions espacées d'une bande sensiblement non élastique en déplacement, qui comprend des moyens pour appliquer de l'adhésif sur des portions discrètes du ruban élastique étiré, des moyens pour délivrer le ruban étiré et la bande en déplacement à un poste d'application du ruban sur la bande, et des moyens pour maintenir au moins une portion du ruban à l'état étiré jusqu'à ce que l'adhésif durcisse, afin de faire adhérer des portions de ruban étiré à la bande, une surface de base étant prévue au poste d'application du ruban élastique, caractérisé par des moyens de coupe qui agissent de manière à s'appliquer simultanément contre le ruban et une portion de la largeur de la bande, sans découper la bande.

12. Dispositif selon la revendication 11, caractérisé en ce que la surface de base est associée à la périphérie d'un tambour rotatif pour recevoir le ruban et la bande superposés, et les moyens de coupe comprennent une arête tranchante et une surface de billot disposés de façon à coopérer l'une avec l'autre, l'arête tranchante ou la surface de billot étant associée à la surface de base.

13. Dispositif selon la revendication 12, caractérisé en ce que l'arête coupante est associée à la surface de base de manière à tourner avec le tambour, et la surface de billot est disposée de manière à coopérer avec l'arête coupante lorsque le ruban et la bande superposés passent entre ces deux éléments.

14. Dispositif selon la revendication 12, caractérisé en ce que la surface de billot est associée à la surface de base de manière à tourner avec le tambour, et l'arête tranchante est disposée de manière à coopérer avec la surface de billot lorsque le ruban et la bande superposés passent entre ces deux éléments.

0 108 173

Fig.1

*Fig.2*

*Fig.1A*

*Fig.3*

fig. 4

fig. 5

_Fig. 6_

202

60

202

200

70

_Fig. 7_

60

70

60

70

_Fig. 8_

60

70

_Fig. 9_

60

70

_Fig. 10_

60

70

_Fig. 11_